# EUROPEAN PATENT APPLICATION

(11) **EP 0 992 251 A1**
(43) Date of publication of application: **12.04.2000**
(21) Application number: 99203237.5
(22) Date of filing: 04.10.1999
(51) Int. Cl.: A61L 27/00, A61L 31/00

(54) **Device for tissue engineering a bone equivalent**

(30) Priority: 07.10.1998 EP 98203389
(71) Applicant: Isotis B.V., 3723 MB Bilthoven (NL)
(72) Inventor: de Bruijn, Joost Dick, 2517 AG The Hague (NL); Magalhaes da Cunha, Antonio Augusta, 4700 Braga (PT); dos Reis, Rui Luis Goncalves, 4200 Porto (PT); da Silva Madureira Mendes, Sandra Claudia, 3572 AL Utrecht (NL)
(74) Representative: Ottevangers, Sietse Ulbe

(57) **Abstract**

The invention relates to a device for tissue engineering a bone equivalent comprising a scaffold material, which scaffold material comprises a matrix based on a destructured natural starch-based polymer. The invention further relates to a process for tissue engineering said bone equivalent, a hybrid structure obtainable by said process, and to the use of said hybrid structure in various surgical treatments.

## Description

The invention relates to a device for tissue engineering a bone equivalent and to a process for tissue engineering said bone equivalent. The invention further relates to a hybrid structure obtainable by said process and to the use of said hybrid structure in surgical procedures.

Surgical procedures related to bone tissue deficiencies vary from joint replacement, bone grafting and internal- fixation, to maxillo-facial reconstructive surgery. From a biological perspective, the ideal material to reconstruct osseous tissues is autogenous bone, because of its compatibility, osteoinductivity, osteoconductivity, and lack of immunologic response. However, the limitations of harvesting an adequate amount of autogenous bone, and the disadvantages of a secondary operation to harvest the autologous bone, make this "ideal" material a far from ideal for many surgical procedures.

Alternatives are other bone-derived materials and man-made biomaterials. The first group concerns allogeneic and xenogeneic bone grafts. A problem is that they exhibit the possibility of disease transfer such as HIV or Hepatitis B, a higher immunogenic response, less revascularisation of the graft and manifest unreliable degradation characteristics.

The second group concerns man-made, alloplastic implant materials, or biomaterials, which are readily available in large quantities. The wide variety of biomaterials that are used in clinical applications can be divided into four major categories: metals, ceramics, polymers and composites, which all have their own characteristics. For load bearing bone replacement, currently only metallic materials are being used. The most interesting alloplastic biomaterials for bone replacement are bioactive or osteoconductive materials, which means that they can bond to bone tissue. Bioactive materials can be found in all four of the above mentioned biomaterials categories and include polymers such as poly(ethylene glycol) poly(butylene terephthalate) copolymers, composites of polymers and calcium phosphate ceramics, such as starch-based polymers and hydroxyapatite composites, calcium phosphate ceramics such as hydroxyapatite and Bioglasses or glass-ceramics.

Compared to autogenous bone, the main disadvantage of biomaterials is that, without added osteoinductive agents such as bone morphogenetic proteins, they are not osteoinductive and therefore do not have the ability to actively induce bone formation. Although this can be overcome by adding osteoinductive growth factors to the materials, difficulties still exist to gradually release these factors from the biomaterial surface over a prolonged time period, which is needed to have a sufficient biological response. A further disadvantage is that there are currently no suitable biodegradable materials available to replace load bearing bone.

This is why another approach for the treatment of osseous defects has to be investigated, which combines cultured autogenous or allogenous cells or tissues with biomaterials, in so-called biomaterial-tissue hybrid structures.

US Patent 5,226,914 discloses a method for treating connective tissue disorders by isolating and culturally expanding marrow-derived mezenchymal stem cells, adhering the, cells onto the surface of a prosthetic device and implanting the prosthetic device containing the culturally expanded cells into the type of skeletal or connective tissue needed for implantation.

US Patent 5,399,665 discloses the synthesis and applications of a hydrolytically degradable polymer useful in biomedical applications involving the interaction of cells with the polymer structure, by coupling peptides to the free amino groups of the polymers.

US Patent 5,041,138 discloses methods and artificial matrices for the growth and implantation of cartilaginous structures and surfaces and the production of bioactive molecules manufactured by chondrocytes. Chondrocytes are grown in culture on biodegradable, biocompatible fibrous matrices until an adequate cell volume and density has developed for the cells to survive and proliferate in vivo, and the matrices are designed to allow adequate nutrient and gas exchange to the cells until engraftment and vascularisation at the site of engraftment occurs.

WO95/03011 discloses a biodegradable prosthetic template of a degradable polymer such as poly(lactic acid) or poly(lactic-co-glycolic acid) with a pore-former such as salt or gelatin, which template may be seeded with osteoblasts. The polymers used are not suitable to replace load-bearing bone and the osteoblasts are highly differentiated cells.

WO96/28539 proposes a composition for growing cartilage or bone consisting of a biodegradable polymeric carrier such as a polyglycolic acid containing mesenchymal stem cells. Mesenchymal stem cells are cells which are pluripotent, i.e. which can differentiate to various tissue types (muscle, cartilage, skin), while the polymers proposed are not suitable to replace load-bearing bone.

These prior art methods involve cells that are grown in materials for the purpose of expansion or proliferation after which the materials containing the culturally expanded cells, are implanted at the site of engraftment. These prior art materials are degradable matrices, either or not designed to couple peptides or biologically active moieties to serve to enhance binding of cells to the polymer, that mainly function as temporary devices for cell attachment. The prior art materials are furthermore generally made from synthetic polymers and they are not suitable to replace load-bearing bone. These prior methods therefore necessitate the production of connective tissues in vivo, while the prior materials function as a carrier for cell attachment and cell growth.

In the Proceedings of the 1998 56^{th} annual technical conference, ANTEC, part 3, Atlanta, GA, USA, at pages 2733-2737, R.L. Reis has proposed to use a blend of native maize starch and either poly(ethylene vinyl alcohol) or cellulose acetate. Native starch, which is in principle unmodified starch, has however been found to be unsuitable for use as a material in the manufacture of scaffolds for tissue engineering bone, particularly load-bearing bane. It has been found that particularly the mechanical properties of native starch blended with polyethylene vinyl alcohol) or cellulose acetate are inadequate. Furthermore, the blends disclosed by Reis based on native starch lack thermoplastic properties, making the formation of scaffolds for tissue engineering of various, often intricate, shapes very complicated.

Accordingly, the present invention aims to provide a device that is based on a polymer of natural origin and which has such good properties, particularly mechanical properties, that it can be used to replace both non-load bearing bone and bone bearing bone, in which cells may be cultured to produce an extracellular matrix. The obtained biomaterial-tissue hybrid structure should be suitable for implantation at a site of engraftment.

Surprisingly, it has been found that this goal is achieved by using a polymeric matrix based on a destructured, natural starch-based material. Thus, the invention relates to a device for bone tissue engineering comprising a scaffold material, which scaffold material comprises a matrix based on a destructured natural starch-based material.

The present invention concerns a device made up from a natural, non-synthetic starch-based polymeric material or polymeric blend that is biocompatible, biodegradable and has mechanical properties similar to the bone it is aimed to replace, that can be used to culture undifferentiated, differentiated, osteogenic or (osteo)progenitor cells that form a bone-like extracellular matrix in vitro, after which the polymer containing the cells and the biological extracellular matrix is placed or implanted at the site of engraftment. The uniqueness about the present invention is twofold. In a first aspect, in contrast to the prior art methods, the material is based on a natural, non-synthetic polymer and exhibits mechanical properties that can be altered to mimic the mechanical properties of the bone it is intended to replace; i.e. non-load bearing and load bearing bone. These mechanical properties may be improved by a cultured, living bone matrix. In the second aspect, undifferentiated, differentiated, osteogenic or (osteo)progenitor cells may be grown in the biodegradable polymeric matrix not only to expand, but to actively produce an extracellular matrix in vitro. Consequently, a hybrid structure encompassing a mechanically strong bioactive, biodegradable non-synthetic polymeric matrix and an already in vitro formed biological extracellular matrix, which adds to the mechanical properties of the material, is produced that can be used for engraftment in osseous defects or at sites where bone is needed. This invented hybrid structure can be seen as a mechanically strong flexible autogenous cultured bone graft, which is unique. Furthermore, the combination of cultured cells and biomaterial is also advantageous in that the cultures cells, already prior, and also after implantation, may give rise to the formation of tissue.

The device of the invention comprises a scaffold material comprising a matrix. This polymeric matrix is based on a destructured natural starch-based material. The destructed natural starch-based material has been described in the prior art for different applications, such as in the packaging industry. Illustrative references are EP-A-0 400 532, EP-A-0 758 669 and EP-A-0 722 980, which are incorporated herein by reference.

The starch on which the polymeric matrix is based, may be obtained from any origin. In principle all starches of natural or plant origin that is composed essentially of amylose and/or amylopectin may be used. The starch can be extracted from various plants, such as for example potatoes, rice, tapioca, maize and cereals, such as rye, oats and wheat. Chemically modified starches and starches of different genotypes may also be used.

An important aspect of the invention is that a destructured starch is used.

The term destructured starch refers to a product in which the starch polysaccharides form a substantially continuous polymeric entangled phase or a substantially completely disordered molecular structure of the granular starch. Destructured starches (DS) are sometime designated thermoplastic starches (TPS) and can be processed using conventional techniques such as extrusion, compression molding, injection molding and blow molding.

One of the most important properties of native starch is its semi-crystallinity. To be able to make a destructurized starch (DS) product, that can be processed by conventional processing techniques such as extrusion or injection moulding, it is necessary to disrupt the granule and melt the partially crystalline nature of starch in the granule. For granular starch the glass transition temperature (T_{g}) is aboe the T_{d} of the polymer chains due to the strong interactions by hydrogen bonding of the chains. Therefore, plasticizers are preferably added to lower the T_{g} beneath the T_{d}. Very important factors that will determine the final properties of DS products are, among others, the type and amount of used plasticizers, the amylose/amylopectin ratio and the molecular weight of the starch (both mainly dependent on the plant of origin), and the final crystallinity of the products. Important examples of plasticizers are water, and several polyols such as glycerol and glycol. Of course, the additives are preferantially fully biodegradable natural or synthetic products.

The production of DS can be achieved by the distruption of granular starch in the presence of a substantial amount of water (preferably more than 10%) and the application of heat and mechanical energy. Preferably, the starting material for the destructuring is starch as it is, without drying beforehand or adding water. The application of heat and mechanical energy will usually be done in an extruder, preferably in a twin-screw extruder, by the action of a thermo-mechanical stress field. The main parameters influencing starch conversion are shear forces, residence time and shear rate, and are defined by the geometry of the extruder as well as by processing variables, such as temperature, screw speed, feed composition and water content. An example of a successful destructurization route involves the heating to a temperature above 120°C , preferably between 140 and 170°C, at low pressure in a single or twin-screw extruder in the presence of destructurizing agents, as indicated below.

The application of unblended DS is limited because of degradation of starch due to water loss at elevated temperatures. Generally for temperatures exceeding 180-190°C, rapid degradation occurs during processing of DS. The behavior of DS is glassy and materials is most suitably processed after the addition of water, other plasticizers or melt flow accelerators. Besides water, several other plasticizers, like polyols (usually with a boiling point of at least 150°C), urea or other chemical compounds (including glycerine polyethylene glycol, ethylene glycol, propylene glycol, sorbitol and mixtures thereof) can be used as destructurizing agents. The plasticizers are preferably used in amounts of 0.05 to 100% of the weight of the starch. Urea is preferably used in addition to another plasticizer in an amount of 2 to 20% of the weight of the starch. Additionally, several other additives (e.g. lubricants) are being used such as lipids, lecithin, fatty acids and glycerol monostearate to improve the flow properties of the DS products.

In order to overcome the difficulties associated to the limited applicability of unblended DS, while the starch is being destructurized in the extruder it is possible to add, together with the plasticizers and other additives, other polymers in order to create biodegradable blends that will confer a more thermoplastic nature to the DS. Other aimed properties are a better resistance to thermo-mechanical degradation, meaning that the blends are more readably processable, have a less brittle nature, and an enhanced resistance to water. The blends produced in this way can be inter-penetrating networks (or not), and be miscible or non-miscible. The thermoplastic polymers used in the blends may include ethylene-acrylic acid, polyvinyl alcohol, ethylene-vinyl and ethylene-vinyl alcohol co-polymers, cellulose acetate and other cellulose derivatives, polycrapolactone, poly(α-hydroxyacids), and mixtures thereof. These thermoplastics may be present in an amount of 15 to 40% of the weight of starch.

The destructured starch described above, as opposed to non-modified, or native, starch, can be used to produce porous scaffolds for bone tissue engeering by a range of methods, including melt based techniques (such as extrusion, injection or compression moulding), by ordering fibres, fibre meshing or producing of open cell foams (for instance by salt leaching, solvent casting or using blowing agents), or radiation based methods. The scaffold material can be shaped to a range of forms, from films to flexible sheets, woven or intertwined fibres or a 3D architecture. The destructurization and blending allowes for the tailoring of the mechanical (ideally bone-matching after reinforcing with bioactive ceramics) and degradation properties of the starch based materials, making the polymers more readably processable, less brittle, and with an enhanced resistance to water (as needed for materials to be implanted in the body).

Thus, the scaffold material may comprise destructured starch, a thermoplastic polymer and a plasticizer. these additional components will be added to the starch prior to the destructurization treatment. The thermoplastic polymer may be chosen from the group of ethylene-acrylic acid, polyvinyl alcohol, an ethylene-vinyl copolymer, a cellulose derivative, polycaprolactone, and mixtures thereof. The thermoplastic polymer may be present in an amount of 0 to 40%, preferably from 15 to 40% of the weight of the starch. The plasticizer may be chosen from the group of water, glycerine, polyethylene glycol, ethylene glycol, propylene glycol, sorbitol and mixtures thereof. Plasticizers belonging to the group of polyols having boiling points of at least 150°C can generally be used. The amount of plasticizer may vary between 0.05 and 100% of the weight of the starch, preferably between 20 and 100% of the weight of the starch.

An important aspect of the polymeric matrix is that it is biodegradable. It preferably is biodegradable to such an extent that it is sufficiently firm to replace load-bearing bone. Further, the biodegrading, after implantation, preferably occurs at such a rate that the mechanical strength that is lost as a result of the biodegradation is substantially replaced by mechanical strength provided by cells growing in the polymeric matrix.

In a preferred embodiment, the scaffold material further comprises a calcium phosphate, a bioactive glass or a bioactive glass ceramic, an adhesive, a bioactive protein, or a combination thereof. These further components of the scaffold material facilitate in vitro and in vivo cell growth, matrix production and the bonding between the device and living cells, e.g. of an extracellular matrix. The scaffold material may be immersed in a calcifying solution, optionally after a chemical or physical surface treatment, for incorporation of a bioactive filler material, such as needle-shaped carbonate-apatite or hydroxyapatite (calcium phosphates, bioactive glasses or glass ceramics). Also, adhesive molecules or bioactive proteins may be added to the material.

The present device may be a non-, partial or fully porous material. Besides osteoconductive properties, the device preferably has an open pore branching network, composed of a biocompatible and ideally biodegradable biomaterial, that is configured in an arrangement that provides for the diffusion of nutrients, oxygen and waste products. The device may be biodegradable or non-biodegradable, dependent on its envisaged application.

Porosity may, among others, be obtained as a result of ordered fibers, fiber meshes (e.g. weaving) or open cell foams (e.g. as a result of salt addition or foaming agents). The scaffold material preferably has the form of an elastic film, a flexible sheet, woven or intertwined fibers or a three dimensional structure. The form chosen will depend on the envisaged application of the device.

The device is preferably a partially or fully porous structure. The pore diameter preferably lies between 50 and 800 µm, more preferably between 200 and 500 µm. Particular preferred is the embodiment, wherein the scaffold material has a pore size gradient. Preferably, the pore diameter increases from 0 to 800 µm from one side of the material to the other.

The mechanical properties of the scaffold material may be obtained by using polymer blends, filler materials processing tools, or combinations thereof. The compressive strength preferably lies between 1 and 280 MPa, the tensile strength preferably lies between 1 and 160 MPa, and the elasticity modulus preferably lies between 0.1 and 40 GPa. These properties may be further improved by the incorporation of living cells into the polymeric matrix of the scaffold material to form a hybrid structure. This hybrid structure is a bone equivalent by which is meant that it has properties similar to the bone of an organism in which the bone equivalent is to be implanted.

The invention further relates to a process for tissue engineering a bone equivalent using a device as described above, wherein living cells are grown in vitro in the scaffold material to produce an extracellular matrix. The living cells may be undifferentiated, differentiated, osteogenic, progenitor, osteoprogenitor cells or combinations thereof.

According to this process, preferably living cells are cultured in a separate medium in which cell growth may take place. Once sufficient cell growth has taken place, the cell suspension is applied to the above described device. In order to obtain a good distribution of the cell suspension throughout the polymeric matrix, this is preferably done at reduced pressure, particularly when a porous polymeric structure is used. The polymeric matrix, with the cell suspension, is subsequently kept in a culture medium for sufficient time for the cells to differentiate and to obtain an extracellular matrix in the polymeric matrix. This second culture medium is preferably chosen such that good conditions are created for cell differentiation to take place.

In a preferred embodiment, the living cells are chosen from the group of soft connective tissue, fibrous tissue, cartilage, muscle tissue, mucous epithelium, urothelium, enaothelium, ligaments, and tendons. Highly preferred living cells used are bone marrow cells originating from the organism in which the desired bone equivalent is to be implanted.

The device preferably possesses osteoinductive properties. These properties will be the results of the formation of a hybrid structure by in vitro formation of an extracellular bone matrix with either osteoinductive proteins, the presence of osteogenic cells, or a combination thereof. In order to accelerate proliferation, differentiation and extracellular matrix production by the cultured cells, osteoinductive factors, growth factors or other biologically active agents may be incorporated into the device. Preferably, these biologically active agents are, after implantation of the hybrid structure into a living organism, gradually released to give rise to a desired biological response.

After the tissue engineering process, a hybrid structure is obtained comprising a polymeric and an extracellular matrix. This structure is equivalent to bone in that it has similar properties. It can be used in a variety of surgical treatments where osseous generation or regeneration is needed. These treatments include all bone defects, of either non-load bearing or load bearing bones, in orthopeadics, maxillofacial surgery, dentistry and other disciplines where osseous (re)generation is required. In a preferred embodiment, the hybrid structure is used for guided tissue regeneration membranes.

The invention will now be elucidated by the following, non-restrictive examples.

### EXAMPLE 1

### IN VITRO CYTOTOXICITY TESTING OF STARCH BASED MATERIALS

### Objective

The objective of these experiments was to obtain data regarding the possible cytotoxic effects of the various starch-based materials.

### Materials

- Blend of corn starch and ethylene vinyl alcohol, 60/40 mol/mol (DSEA)
- DSEA + 10% HA filler (HA = hydroxyapatite)
- DSEA + 20% HA filler
- DSEA + 30% HA filler
- DSEA + 40% HA filler
- DSEA + 30% HA filler + 1% coupling agent 1 (neoalkoxy titanate)
- DSEA + 30% HA + 1% coupling agent 2 (neoalkoxy zirconate)
- Porous DSEA + 5% blowing agent
- Porous DSEA + 10 % blowing agent
- Porous DSEA + 20% blowing agent

DSEA was prepared by starting from a composition containing:
- 63 % by weight of undried GLOBE 03401 CERESTAR (trademark) starch with a water content of 11%;
- 25% by weight of glycerine;
- 7% by weight of urea;
- 5% by weight of the Dow Chemical copolymer EAA 5981 containing 20% of acrylic acid.

The components were supplied from a Licoarbo DC-10 batcher to a Baker Perkins MPC/V-30 extruder. The extruder was constituted by a two-screw unit divided into two regions, with a screw diameter of 30 mm and a screw length/diameter (L/D) of 10:1, and connected to a single-screw extruder press with a capillary head and a screw having a diameter of 30 mm and an L/D ratio of 8:1, divided into three regions. The capillary nozzle used has a diameter of 4.5 mm.

The extrusion temperature was 140°C.

The extrusion obtained was pelletized without problems.

60% of destructured starch pellets and 40% by weight of Clarene R20 ethylene/vinyl alcohol copolymer were extruded at 160°C in the same extruder. The final blend was blown at 160°C in a HAAKE Reomex extruder, model 252, with an L/D ratio of 19, a screw diameter of 19 mm, and a compression ratio of 1:3, and with the screw revolving at 40 rpm.

The product obtained was characterized by a melting point of 135°C and a glass transition temperature of 70°C.

In the cases where DSEA was used in combination with hydroxy apatite (HA) and optionally a blowing and/or coupling agent, these additives were introduced simultaneously with the ethylene/vinyl alcohol copolymer in the indicated amounts.

In all the tests performed, UHMWPE and latex rubber were used, respectively as negative and positive controls. Prior to cytotoxicity testing, the materials were sterilised with ethylene oxide and subsequently handled in a sterile manner.

### Methods

In order to assess the short term cytotoxicity of the test materials two cell culture methods, according to ISO/EN 109935 guidelines, were used: MEM extraction test (72 h) and MEM-MTT tests (72 h), both with a 24 h extraction period. The MEM test allows for the quantitative measurement of cell death and proliferation and the qualitative evaluation of cell adhesion and morphology. The MEM- MTT assay allows the quantification of the biosynthetic activity

### Cell culture

In these tests mouse fibroblastic lung cells (L929 cell line) were used. L929 cell line was grown as monolayer culture in, Dulbecco's modified eagle's medium (DMEM) supplemented with 10% Foetal bovine serum (FBS), 1% Fungizone and 0.5% Penicillin streptomycin. The cell culture atmosphere was 5% CO₂ in air.

### MEM extraction test

In all tests the ratio material outer surface/extraction fluid volume was constant and equal to 3 cm²/ml. For the short-term cytotoxicity trials, the test materials were extracted during 24h at 37°C. and the extraction fluid used was the complete cell culture medium. For the long-term cytotoxicity tests the extraction procedure was very similar, however the extraction fluid did not contain FBS, which was only added when the extraction period was over. In these tests the materials were extracted for periods of 4, 10, 20 and 35 or 40 days. In the beginning of the tests the culture medium was replaced by the same amount of extraction fluid and the reaction of the cells was evaluated, after 24, 48 and 72 hours, for confluence of the monolayer, degree of floating cells and changes in morphology. After 72 hours testing, the percentage of growth inhibition was determined by cell counting using a cytometer. All the measurements were corrected for the negative control.

### MEM-MTT assay

This assay followed the same extraction and culture procedure described previously for the MEM test. After 72 hours testing, a MTT solution was prepared (1mg MTT/ml of working solution). After adding the solution to the plates, they were incubated for 3 hours at 37°C, after which time the MTT solution was removed and replaced by isopropanol to lyse the cells. The optical density (OD) was then measured in a photospectrometer, at 570 nm, with a background correction of the OD at 690 nm. The mean OD570mm value obtained for the negative control was standardized as 0% of metabolism inhibition.

### Results

### Short-term MEM extraction tests

The final results from the short term MEM extraction tests are shown in table 1. In these tests starch-based materials have demonstrated to be non-cytotoxic. Cells in contact with all test materials extracts did not show any signs of death and revealed normal morphology.

**Table 1**

| Results from the short-term MEM extraction tests (after 72h testing) | | | |
|---|---|---|---|
| Extract | Growth inhibition (%) | Cytotoxic effect | Result |
| DSEA | 2.80 | None | Pass |
| DSEA + 10% HA | 62.2 | Slight | Pass |
| DSEA + 20% HA | 41.3 | Slight | Pass |
| DSEA + 30% HA | 59.8 | Slight | Pass |
| DSEA + 40% HA | 61.1 | Slight | Pass |
| DSEA + 30% HA + 1% coupling agent 1 | 43.3 | Slight | Pass |
| DSEA + 30% HA + 1% coupling agent 2 | 42.1 | Slight | Pass |
| DSEA + 5% blowing agent | 35.6 | Slight | Pass |
| DSEA + 10% blowing agent | 38.7 | Slight | Pass |
| DSEA + 20% blowing agent | 36.3 | Slight | Pass |

### MEM-MTT assay

In the MEM - MTT tests the cell line, when in the presence of the test materials extracts, produced relatively large amounts of blue formazan. As it can be observed in table 2 neither of the test materials showed significant inhibition of cell metabolism.

**Table 2**

| Results from the MEM-MTT tests (after 72h testing) | | |
|---|---|---|
| Extract | Inhibition of cell metabolism (%) | Result |
| DSEA | 0.00 | Pass |
| DSEA + 10% HA | 0.00 | Pass |
| DSEA + 20% HA | 0.00 | Pass |
| DSEA + 30% HA | 0.00 | Pass |
| DSEA + 40% HA | 0.00 | Pass |

### Long term MEM extraction tests

The results from the long-term MEM extraction tests are shown in table 3 and 4. For all extraction periods the extracts had no significant effect on cell morphology, spreading and growth.

**Table 3**

| Results from the long term MEM extraction tests (after 72h testing) | | | | | | |
|---|---|---|---|---|---|---|
| | Growth Inhibition (%) | | | | | |
| Extract | 4 days extraction | 4 days extraction | 20 days extraction | 40 days extraction | Cytotoxic effect | Result |
| DSEA | 3.9 | 2.2 | 3.4 | 10.1 | None | Pass |
| DSEA + 30% HA | 22.5 | 28.5 | 27.7 | 35.8 | Slight | Pass |

**Table 4**

| Results from the long term MEM extraction tests (after 72h testing) | | | | | | |
|---|---|---|---|---|---|---|
| | Growth Inhibition(%) | | | | | |
| Extract | 4 days extraction | 4 days extraction | 20 days extraction | 35 days extraction | Cytotoxic effect | Result |
| DSEA | 0.00 | 1.8 | 6.5 | 11.3 | None | Pass |
| DSEA + 10% HA | 3.1 | 3.1 | 10.3 | 23.1 | None | Pass |
| DSEA + 20% HA | 4.6 | 4.9 | 12.5 | 33.9 | Slight | Pass |
| DSEA + 30% HA | 7.3 | 8.6 | 17.4 | 35.3 | Slight | Pass |
| DSEA + 40% HA | 12.1 | 12.8 | 21.1 | 35.7 | Slight | Pass |

### Conclusions

In conclusion it can be stated that the starch-based materials have not shown relevant toxicity in both short and long term in vitro testing.

### EXAMPLE 2

### IN VIVO EVALUATION OF STARCH BASED MATERIALS

### Objective

The objective of this experiment was to obtain data on the starch based materials in vivo performance. The tissue reactions in both cortical bone and muscle were examined.

### Materials

- Blend of corn starch and ethylene vinyl alcohol, 60/40 mol/mol (DSEA) prepared as described in Example 1; cylindrical bars of 0.5 cm in diameter and 0.7 cm in length
- DSEA + 50% HA; cylindrical bars of 0.5 cm in diameter and 0.7 cm in length

### Methods

In vivo implantation tests were performed intra-cortical and intramuscularly, on Dutch milk goats, according to good laboratory practices (GLP) regulations. After 6 and 12 weeks survival periods the biocompatibility, biodegradation, bone contact and bone bonding ability of the implanted materials were evaluated. The characterisation of the explanted samples included, light microscopy for histological evaluation, histomorphometry and scanning electron microscopy.

### Experimental design and surgical procedure

Prior to surgery the goats were weighed and amphicillin 20% (2 ml/50 kg body weight) was administrated by subcutaneous injection. The surgery was performed under general inhalation anaesthetic. The implantation areas were shaved and disinfected with iodine. For the intra-cortical implantation, the femora were exposed by a lateral skin incision and blunt dissection. Four holes were drilled in the lateral cortex, each defect was created to a final diameter of 5 mm. Implants were inserted randomly and press-fit in the holes by gentle tapping (8 implants per material and per implantation period). The muscle fascia and skin were closed using vicryl sutures. For the intramuscular implantation an incision was made in the skin, after which the muscle fascia was exposed. Subsequently, an incision is made in the muscle followed by the preparation of a pocket. After placing randomly the implants (4 per material / and per implantation period), the muscle and skin were sutured with vicryl. Respectively 6 and 12 weeks post-operatively, the animals were sacrificed using an intravenously administered overdose of thiopental and potassium chloride.

### Histology

After explantation the samples were placed Karnovsky's fixative (5% paraformaldehyde, 4,5% glutaraldehyde, pH=7.4). Subsequently, the intra-cortical implants were isolated 'en bloc' and placed in fresh Karnovsky's fixative. After one-week fixation, both intramuscular and intra-cortical implants were dehydrated in graded series of ethanol and subsequently embedded in methyl methacrylate (MMA). Undecalcified sections from all samples were cut parallel to the longitudinal axis of the bone, using a histological diamond saw. The sections, approximately 10 µm thick, were stained with methylene blue and basic fuchsin and examined in a light microscope.

### Histomorphometry

To measure the percentage of bone contact and bone remodelling at the bone/implant interface, quantitative analysis of 3-4 sections of each intra-cortical implant was performed.

### Scanning electron microscopy

After preparation of the light microscopical sections, the remaining MMA blocks were polished with 4000 grit silicon carbide sandpaper and coated with a layer of carbon. Samples were examined in a Philips S525 scanning electron microscope both in secondary and back-scattered modes, working at an accelerating voltage of, respectively 20 kV and 15 kV.

### Results

All animals recovered rapidly from the surgical procedures, without post-operative complications. In the present study the morphological appearance of the interface was analysed for both intramuscular and intra-cortical implants. During the entire implantation periods, no chronic inflammation or tissue necrosis could be detected. After 6 weeks survival, around the intramuscular implants and in the medullary cavity of DSEA and DSEA + 50% HA intra-cortical implants, a very thin layer with a few inflammatory cells, mainly foreign body giant cells, was formed. However, these cells were restricted to regions very near to the implant surface and the thickness of this cellular reaction did not increase with the longer implantation period. Considering the bone reactions to both implantation procedure and presence of implants, after 6 weeks survival, at a light microscopical level, contact between both materials and bone was seen over large areas. The intervention of fibrous tissue was only encountered in areas where bone was remodelling, however, for this implantation period, the cortical remodelling process was just in the initiation stage. After 12 weeks implantation, the cortical bone surrounding both types of implant materials showed extensive areas of bone reaction. New bone with mature osteocytes was formed and remodelling lacunae with osteoblasts were also detected. Sometimes a contact between implants and the old lamellar bone was still observed. Concerning the stability and integrity of the implant materials, for both implantation periods, in the medullary cavity of the intra-cortical implants as well as in all intramuscular implants, a de-coloration of the outer surface together with a swelling pattern was always observed. SEM analysis also pointed out that both materials went through morphological changes during implantation, especially in their outer regions.

### Histomorphometry

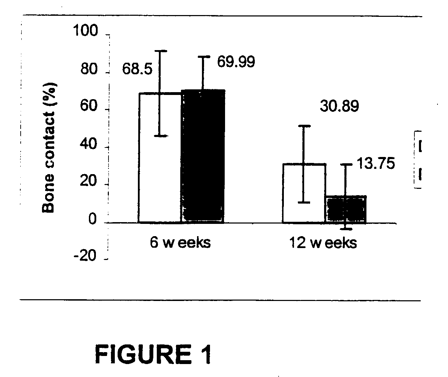

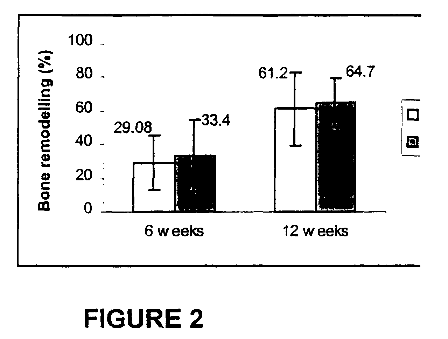

The percentage of bone contact and remodelling after 6 and 12 weeks survival periods is illustrated in figures 1 and 2. Figure 1 depicts the percentage of bone contact on the intra-cortical implants as a function of time. Figure 2 depicts the percentage of bone remodelling on the intra-cortical implants as a function of time. (In both figures, the white column refers to DSEA, and the gray column refers to DSEA + HA).

The earlier survival time revealed a relatively high degree of bone contact (70%) in the cortical region and the differences between DSEA and DSEA + 50% HA for the two measured parameters were not significant. For both implant materials, after 12 weeks implantation, bone apposition significantly decreased for less than half of the initial value. Furthermore, at this survival time, the DSEA material exhibited higher bone contact when compared to the composite material. On the contrary, the bone remodelling percentages were approximately the same for both materials.

## Claims

1. A device for bone tissue engineering comprising a scaffold material, which scaffold material comprises a matrix based on a destructed natural starch-based polymer.

2. A device according to claim 1, wherein the starch is destructured by heating to a temperature above 120°C.

3. A device according to claim 2, wherein the destructurization is performed in the presence of a destrucurization agent chosen from the group of urea, and a polyol.

4. A device according to claim 3, wherein the destructurization agent is present in an amount of from 2 to 30% of the weight of the starch.

5. A device according to any of the preceding claims, wherein the scaffold material further comprises a thermoplastic polymer and a plasticizer.

6. A device according to claim 5, wherein the thermoplastic polymer is chosen from the group of ethylene-acrylic acid, polyvinyl alcohol, an ethylene-vinyl copolymer, a cellulose derivative, polycaprolactone and mixtures thereof.

7. A device according to claim 5 or 6, wherein the plasticizer is chosen from the group of water, glycerine, polyethylene glycol, ethylene glycol, propylene glycol, sorbitol and mixtures thereof.

8. A device according to any of the preceding claims, wherein the scaffold material further comprises a calcium phosphate, a bioactive glass or a bioactive glass ceramic, an adhesive, a bioactive protein, or a combination thereof.

9. A device according to any of the preceding claims, wherein the scaffold material is partially or fully porous.

10. A device according to claim 9, having pores having a diameter of from 50 to 800 µm, preferably from 200 to 500 µm.

11. A device according to claim 9 or 10, having a pore size gradient ranging from a pore size of from 0 to 800 µm.

12. A device according to any of the preceding claims, wherein the scaffold material is an elastic film, a flexible sheet, woven or intertwined fibers or a three dimensional structure.

13. A device according to any of the preceding claims having a compressive strength between 1 and 280 MPa, a tensile strength between 1 and 160 MPa, and an elasticity modulus between 0.1 and 40 GPa.

14. A process for tissue engineering a bone equivalent using a device according to any of the preceding claims, wherein living cells are grown in vitro in the scaffold material to produce an extracellular matrix.

15. A process according to claim 14, wherein the living cells are chosen from undifferentiated, differentiated, osteogenic, progenitor, osteoprogenitor cells and combinations thereof.

16. A process according to claim 15, wherein the living cells are selected from the group of soft connective tissue, fibrous tissue, cartilage, muscle tissue, mucous epithelium; urothelium, endothelium, ligaments, and tendons.

17. A hybrid structure obtainable by a process according to any of the claims 14-16 comprising a device according to any of the claims 1-13 and a bone-like extracellular matrix comprising living cells.

18. The use of a hybrid structure according to claim 17 in surgical treatments of bone defects in orthopaedics, maxillofacial surgery, and dentistry.

19. The use of a hybrid structure according to claim 17 for guided tissue regeneration membranes.

20. The use of living cells for in vitro forming an extracellular matrix in a device according to any of the claims 1-13, thereby improving the mechanical strength of said device.
